# EUROPEAN PATENT SPECIFICATION

(11) **EP 2 195 418 B1**
(45) Date of publication and mention of the grant of the patent: **12.12.2012**
(21) Application number: 07827735.7
(22) Date of filing: 02.10.2007
(51) Int. Cl.: C12N 7/02

(54) **PROCESS AND SYSTEM FOR THE INDUSTRIAL SCALE PURIFICATION OF BACTERIOPHAGES INTENDED FOR BACTERIOPHAGE THERAPY**
VERFAHREN UND SYSTEM ZUR GROSSTECHNISCHEN AUFREINIGUNG VON FÜR EINE BAKTERIOPHAGENTHERAPIE VORGESEHENEN BAKTERIOPHAGEN
PROCÉDÉ ET SYSTÈME DE PURIFICATION À L'ÉCHELLE INDUSTRIELLE DE BACTÉRIOPHAGES DESTINÉS À UNE THÉRAPIE PAR BACTÉRIOPHAGES

(43) Date of publication of application: 16.06.2010
(73) Proprietor: PTC Phage Technology Center GmbH, 59199 Bönen (DE)
(72) Inventor: LEHNHERR, Hansjörg, 3014 Bern (CH); BARTSCH, Reinhard, 79249 Merzhausen (DE)
(74) Representative: Kalkoff & Partner
(86) International application number: PCT/IT2007/000684
(87) International publication number: WO 2009/044414

(56) References cited:
- EP-A- 1 736 538
- EP-A- 1 780 269
- WO-A-2006/052302
- US-B1- 6 261 823
- SMREKAR ET AL: "Purification and concentration of bacteriophage T4 using monolithic chromatographic supports" JOURNAL OF CHROMATOGRAPHY B: BIOMEDICAL SCIENCES & APPLICATIONS, ELSEVIER, AMSTERDAM, NL, vol. 861, no. 2, 7 July 2007 (2007-07-07), pages 177-180, XP022432657 ISSN: 1570-0232

## Description

The present invention deals with a process and a system for the industrial scale purification of bacteriophages intended for bacteriophage therapy.

As an adaptation to the selective environment they encounter, many pathogenic bacteria have become resistant to a wide range of antibiotics [9]. Alternative forms of treating such resistant bacteria are thus in high demand. Bacteriophage therapy is one of the alternatives considered today. Worldwide, the interest in bacteriophage therapy is on the rise, after it has been dormant in the Western World, for almost 60 years [6]. Past applications of bacteriophage therapy have been hampered by the inability to purify bacteriophage preparations in order to remove exo- and endotoxins, as well as to preserve the biological activity of the bacteriophages [8]. Current methods to produce and purify bacteriophages are derived from laboratory methods and are not suitable for large scale preparations. The present invention relates to the large scale production and purification of bacteriophage preparations in order to combat infectious diseases, especially, but not exclusively, when the bacteria causing these diseases are resistant to antibiotics. It describes a method to produce, on an industrial scale, bacteriophage compositions that are highly concentrated and free of any toxic remnants (bacterial debris, bacterial endo- and exotoxins), that are the byproduct of the production process.

The use of bacteriophages to fight bacterial infections has already been proposed by d'Hérelle, the co-discoverer of the bacteriophages [2]. However, while some of the early applications proved to be successful, others failed, in retrospect mainly due to the lack of knowledge about the interactions between bacteriophages and their host bacteria [11]. With the advent of the antibiotic area any attempts to establish bacteriophages as antimicrobial agents were abandoned in Western medicine. On the other hand, bacteriophages were widely used in the former Soviet Union and Poland, but unfortunately, most of the Eastern studies demonstrating the efficacy of bacteriophage therapy do not meet the standards of our current medical systems. However, bacteriophages served as major study tools in the field of molecular biology in both East and West, and thus have been intensively investigated in many laboratories over the last eighty years [1]. The knowledge gained from this basic research should now prove useful to prepare improved bacteriophage preparations that will fulfil the strict criteria required for both animal and human applications [7, 11].

In the present invention, bacteriophages are amplified in large-scale fermenters or alternatively on semi-solid medium (see US patent application 2007/001001), producing hundreds of litres of solution enriched in bacteriophages, but contaminated with bacterial debris, toxins and components of the bacterial growth medium. Such a solution is then passed through a series of filters in order to separate the bacteriophages from these contaminating substances. In the process the bacterial growth medium containing the bacteriophages is substituted by a solution suitable for packaging and long term storage of the bacteriophages.

The present invention can be used to purify any bacteriophage in large scale, resulting in preparations that can be used directly as disinfecting agents or medical products for both animal and human use.

Bacteriophages are omnipresent in the environment. Methods to isolate and enrich new bacteriophages with desired host ranges are thus known to those skilled in the art [4, 5, 10]. Theoretically it is possible to isolate bacteriophages that grow on any bacterial pathogen known. However, not all bacteriophages found in nature are also suitable for practical applications. A precise genetic characterization of the isolated phages will be essential to select those that destroy their host bacteria with high efficiency and do not transfer any unwanted traits [8]. To identify those bacteriophages most suited for a specific task is a skill in itself (see for example WO 2004/004495). Also, various methods to improve natural isolates or modify their properties exist, as described in US patents 5811093 or 7087226.

Usually bacteriophages are grown in liquid cultures using fermenters of various sizes [3]. Alternatively bacteriophages can also be grown on semi-solid medium as disclosed in US patent application 2007/0010001. However, either of these two methods creates equally large volumes of liquid phage-containing solutions that need to be purified for further use. Different methods for the purification of virus-like-particles and viruses, especially of bacteriophages have been described for example in EP 1736 538 A, EP 1780 269 A and by Smrekar et al. [12].The current standard laboratory procedure, as outlined below, follows a work intensive, multi-step protocol and thus is only suitable for smaller volumes of liquid.
1. The bacteriophage-containing solution is cleared by centrifugation.
2. Polyethylene glycol is added to the liquid in order to precipitate the bacteriophages. Polyethylene glycol is a non-specific, reversible crosslinker that precipitates all the proteins present in the solution. The precipitation process takes approximately 12-18 hours at 4 degrees centigrade.
3. Precipitation of the bacteriophages by centrifugation.
4. Resuspension of the precipitated bacteriophages in the minimal amount of a suitable liquid. During this step the bacteriophages are usually concentrated about 50 fold.
5. This solution is then carefully layered onto a CsCl₂-density gradient and processed by centrifugation in an ultracentrifuge. In this step the bacteriophages are separated from other proteins and impurities according to their specific density. This process takes approximately 24 hours.
6. Isolation of the bacteriophages using a syringe with a sharp-tipped needle. This process requires a steady hand and some experimental skill.
7. Removal of the CsCl₂-solution by dialysis (12 to 24 hours) against a buffer of choice.

While this procedure results in highly pure bacteriophage preparations, it appears obvious that it cannot be applied to purify bacteriophages on an industrial scale. Not only is the process time consuming, but it requires an inordinate amount of manual work and is not cost effective either.

In Georgia and the former Soviet Union, bacteriophages were not purified to this extent [3]. Usually bacteriophage solutions only were filter-sterilized and then directly packaged for applications. However, such a procedure does not remove a large number of bacterial proteins, among them toxins and almost all the components of the bacterial growth medium, making such solutions unsuitable for present applications.

The above and other objects and advantages of the invention, as will appear from the following description, are obtained with a process and a system as respectively disclosed in the independent claims. Preferred embodiments and non-trivial variations of the present invention are the subject matter of the dependent claims.

The present invention will be better described by some preferred embodiments thereof, provided as a non-limiting example, with reference to the enclosed drawings, in which:
- Figure 1 is a schematic view of a preferred embodiment of the system to which the process of the present invention can be applied;
- Figure 2 is a schematic view of the first part of another preferred embodiment of the system to which the process of the present invention can be applied; and
- Figure 3 is a schematic view of the second part of another preferred embodiment of the system to which the process of the present invention can be applied.

With reference to the Figures, preferred embodiments of the process and system of the present invention are shown and described. It will be immediately obvious that numerous variations and modifications (for example related to shape, sizes and parts with equivalent functionality) can be made to what has been described, without departing from the scope of the invention as appears from the enclosed claims.

The present invention relates to a novel purification method that is based on a series of filtration steps that require no manual input, is thus suitable for large scale, industrial production and results in bacteriophage compositions that are highly pure and essentially free of any residual toxic remnants. These purified bacteriophage preparations can then be packaged in any form suitable for their appropriate agricultural or clinical applications.

On average bacteriophages have a diametre of 50 to 100 nanometres and are 100 to 300 nanometres long. They are composed of proteins that form a protective shell around the genetic material. This protective shell contains delicate structures in the nanometre range, which, when broken, result in the loss of the biological activity of the bacteriophages. Special care has thus to be taken in order not to damage the bacteriophages during the purification process.

The procedure for producing and purifying can be divided into the following steps.

### Step 1. Amplification of bacteriophages.

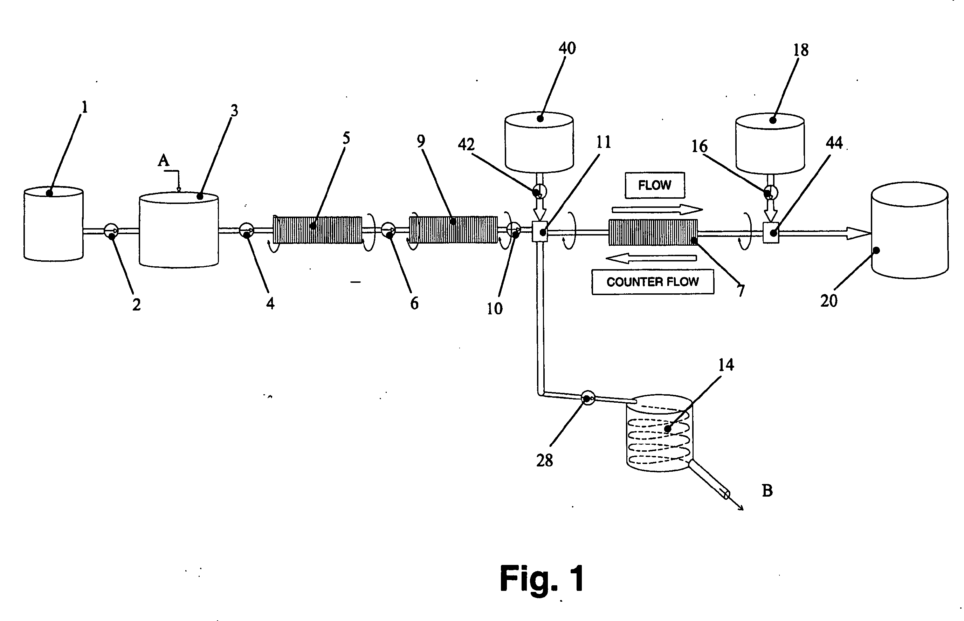

Procedures to produce large numbers of bacteriophages are known to those skilled in the art. Normally host bacteria are allowed to grown in a fermenter 1 in order to reach high cell densities. Another fermenter 3 containing such bacteria supplied by a pump unit 2 is then seeded with high or low numbers of bacteriophages (arrow A in Fig. 1) depending on whether a single step amplification or a multistep amplification should take place. Alternatively bacteriophages can also be grown on semi-solid medium as disclosed in US patent application 2007/0010001. Depending on the bacteriophage to be amplified and the host bacterium in use, either of these methods results in equally large volumes of bacteriophage preparations containing 10⁹ to 10¹² bacteriophages per millilitre. In this invention a setup using two fermenters 1, 3 is proposed, which will be immediately advantageous when more than one bacteriophage has to be purified.

### Step 2. Prepurification.

The goal of this first purification step is to remove contaminants that are larger than the bacteriophages from the bacteriophage preparations. After the amplification step that results in the destruction of most of the host bacteria, the bacteriophage preparations are contaminated with intact bacterial cells, bacterial cell wall fragments, bacterial lipids in membrane vesicles of various sizes, bacterial proteins, among them exo- and endotoxins and various components of the bacterial growth medium (salts, sugars, proteins). This solution is rich in solids and highly viscous. The state of the art purification scheme using polyethylene glycol precipitation and density centrifugation (as described above) is work intensive and not suitable for large volumes. Traditional filtration techniques, like the standard sterile filtration using filters with 200 nm pores, are very inefficient, as they quickly clog when faced with such highly viscous solutions. As a result large aggregates are forming, which trap the bacteriophages and drastically reduce the yield. In this invention, the use of a crossflow rotation filtration system with two filtering units 5, 9 is described, but a single filtering unit 9 (or more than two filtering units) could instead be used, however, with an increased risk of clumping. The bacteriophage preparation is pumped, with a pump unit 4, directly from the fermenter 3 to the filtration unit 5, as shown in Fig. 1, and using low pressures (1 bar) the pump unit 4 passes the solution through a Teflon filter 5, that has a pore size of ≈ 1000 nanometres and rotates with the speed of 400 rotations per minute. Small particles, among them the bacteriophages, pass through the membrane as filtrate, while intact bacterial cells, large bacterial cell wall fragments and bacterial lipids in large membrane vesicles stay behind in the retenate. The low pressure and the rotation of the filter 5 discs prevent large particles from blocking the filter pores. The shearing forces occurring during such a filtration step are relatively low and do not affect the viability of the bacteriophages.

The filtrate of the first filtration unit 5 then serves directly as the feed for a second filtration unit 9, being fed by a pump unit 6. This second filtration unit 9 has an equivalent setup, but the pore size of the Teflon filter is reduced to ≈ 200 nanometres. Again the bacteriophages, bacterial proteins, sugars and salts pass this second filter, while residual bacterial debris are held back in the retenate. The shearing forces occurring during this second filtration step are already considerable, but experiments showed that more than 95% of the bacteriophages manage to pass such a filter intact. Other filter systems could be used, but with increased risk of clumping and thus lower yield.

### Step 3. Purification.

The .filtrate from the prepurification is used as feed for a third filtration unit 7 through a pump unit 10. The goal of this step is to clear the bacteriophage preparations from contaminants that are smaller than the bacteriophages. This is achieved by passing the solution over a filter 7 with a pore size of ≈ 60 nanometres. The bacteriophages are kept in solution in the retenate, while small proteins, among them the toxins, sugars and salts pass the filter and can be discarded and collected in a collecting tank 20. In a regular filtration, little concern is given to the fate of the retenate, as the filtrate is in general the product. Here, however, the bacteriophages are retained in the retenate and special care has to be taken in order to prevent them from clumping or being inactivated by shearing forces. State of the art crossflow filtration systems are not suitable for such a process, as the shearing forces created by a flow over a filter with a pore size of ≈ 60 nanometres are in a range sufficient to inactivate bacteriophages. Thus, in this invention, a milder form of filtration, is described. Using very low pressure (0.2. bar), the bacteriophage preparations are passed through a slowly rotating ceramic filter 7 (200 rotations per min). The low pressure and the rotation of the filter 7 prevent the bacteriophages from coagulating on the filter surface.

Once a batch of the phage preparation is processed, the feeding pump 10 is turned off and a second pump 42 passes a cleaning solution, stored in a vessel 40, through a group of valves 11 and into the filter 7. This washing step with cleaning solution guarantees that no components of the original bacterial growth medium are present in the final product, while still keeping the bacteriophages in solution in the retenate.

### Step 4. Elution.

After the cleaning, a third pump 16 at the back of the filtration unit 7 is activated and pumps a storage solution, contained in a vessel 18, through a group of valves 44, in the opposite direction ("COUNTERFLOW" direction in Fig. 1), using an impulse pressure. Otherwise the same conditions as during step 3 are used. In this storage solution, the highly pure bacteriophages are thus eluted from the rotation filter 7. The impulse pressure is used to remove potential deposits from the pores and the surface of the filter. Using appropriate amounts of storage solution to elute, provides an easy and safe mean to adjust the final concentration of the bacteriophages and thus guarantees a standardized high quality end product.

The purified bacteriophage solutions exiting the filter 7 pass through the group of valves 11 and are pumped, through a fourth pump unit 28 (such pump unit 28 is optional, namely its function can be performed, for example, by the pump unit 42), into an intermediate storage container 14. From the intermediate storage container 14, the bacteriophage solutions can then be packaged directly, either in liquid form or dried after lyophilization, following a normal working flow (arrow B in Fig. 1.).

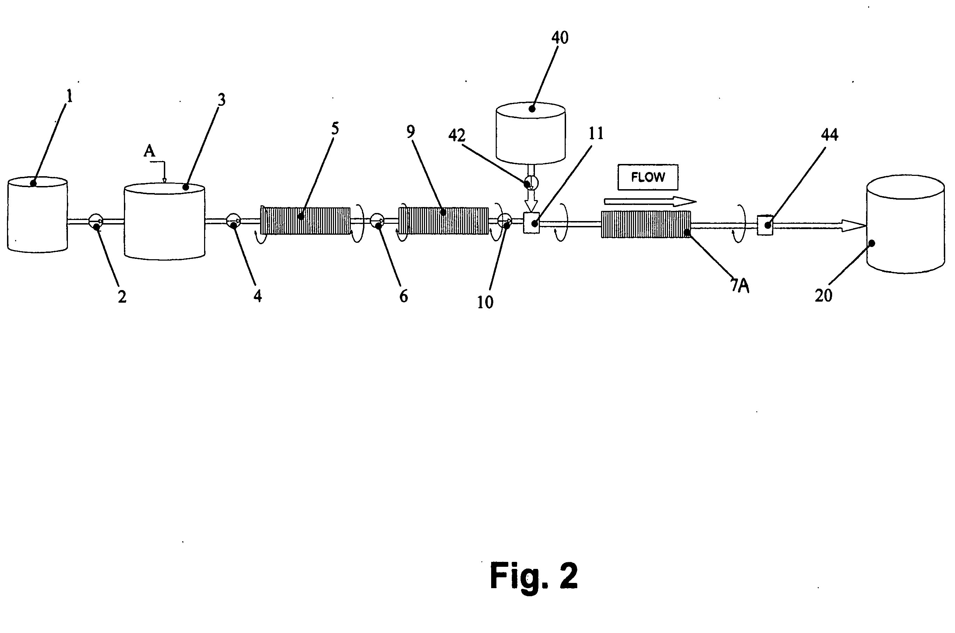

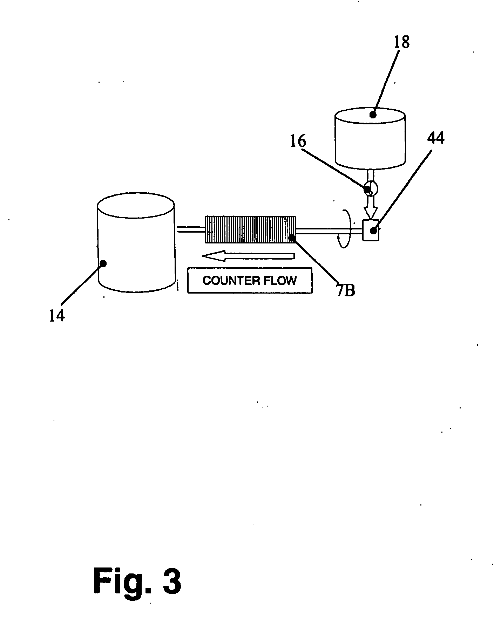

The ideal filtration process is described in Figure 1, with a fully automated workflow, resulting in highly purified bacteriophages, with essentially no losses during the process. However, depending on the production conditions, it might be necessary to separate the "back flow" filtration step from the rest of the process, in order to gain more flexibility. Thus, Figures 2 and 3 show a second embodiment of the system of the invention. The same designation references are kept for the parts with similar or identical functionality.

The main difference between the system of Fig. 1 and the system of Fig. 2 and 3 is that there are two identical, but separate rotary filters 7A, 7B, one of which performs the operation designated with "FLOW" in Fig. 2 and the other one of which performes the operation designated with "COUNTERFLOW" in Fig. 3; while the rotary filter 7A in Fig. 2 is connected to the collecting tank 20 for wastes, the rotary filter 7B in Fig. 3 is connected to the vessel 18 with the storage solution for counterflow, and to the intermediate storage tank 14 for the final solution of purified bacteriophages.

As another possible embodiment of the inventive system, derived again from Figures 2 and 3, the rotary filter 7 could be again only one, as in the first embodiment, shown in Figure 1, but it could be operated separately in two different steps, one similar to Figure 2 where the solution moves along the "FLOW" direction, and another one similar to Figure 3, where the solution moves along the "COUNTERFLOW" direction, obviously performing the two separate steps described above for these two directions and operating steps.

Especially when entire groups of fermenters have to be processed simultaneously, it might be necessary to quickly regenerate the filter system. By removing the rotary filter unit 7A, 7B, rinsing and inserting a fresh filter unit, the entire filtration system as outlined in Figure 2 will be available to process the content of a second fermenter without delay. The removed filter unit 7A, 7B, containing the purified bacteriophages, can then be processed in a separate elution unit, as outlined in Figure 3. The filtration system as outlined in Figure 1 will be designed in order to allow a choice between the two embodiments of the invention.

The systems shown in the Figures are obviously only examples of the different systems that can be used to practice the process of the invention. For example, the filters 5 and 9 could be crossflow rotary filters as shown in the Figures, or could be traditional filters adapted to perform the same filtering functions. Moreover, such filters could be one, two or more, according to the desired filtration task.

Moreover, the intermediate storage tank 14 could be the one shown in Figure 1, where the solution of purified bacteriophages is subjected to a spiral rotation or swirling adapted to obtain an homogeneous distribution of bacteriophages. Alternatively, the intermediate storage tank 14 could be the traditional one shown in Fig. 3 (that can obviously be used also in the system of Fig. 1) where the final solution of purified bacteriophages is not subjected to rotation or swirling.

### List of References

1. Calendar, R. 2006. The Bacteriophages. Oxford University Press, NY.
2. **d'**Hérelle, F. 1926. The Bacteriophage and its Behavior. Willliams and Wilkins, Baltimore, Md.
3. Hauler, T. 2003. Gesund durch Viren. Ein Ausweg aus der Antibiotika-Krise. Piper Verlag GmbH, Munched.
4. Hoff, J. C. and C. H. Drake. 1962. Simplified method for isolation and purification of bacteriophages. J.Bacteriol. 83:929-92b.
5. Hook, A. E., D. Beard, A. R. Taylor, D. G. Sharp, and J. W. Beard. 1946. Isolation wand characterization of the T2 bacteriophage of Escherichia coli. J.Biol.Chem. 165:241-258.
6. Kutter, E. 1997. Phage therapy: Bacteriophage as antibiotics. http://www.evergreen.edu/user /T4/PhageTherapy/Phagethea.html .
7. Lehnherr, H. 2006. Bacteriophage P1, p. 351-364. In R. Calendar (ed.), The Bacteriophages. Oxford University Press, NY.
8. Merrill, C. R., D. Scholl, and S. Adhya. 2006. Phage Therapy, p. 725-742. In R. Calendar (ed.), The Bacteriophages. Oxford University Press, NY.
9. Neu, H. C. 1992. The crisis in antibiotic resistance. Science 257:1064-1073.
10. Putnam, F. W., L. M. Kozloff, and J. C. Neil. 1949. Biochemical studies of virus reproduction. I. Purification and properties of Escherichia coli bacteriophage T6. J.Biol.Chem. 179:303-323.
11. Summers, W. C. 2001. Bacteriophage therapy. Annu. Rev. Microbiol. 55:437-451.
12. Smrekar, F. Ciringer, M., Peterka, M., Podgornik, A., Strancar, A. 2007. Purification and concentration of bacteriophage T chromatographic supports. J. Chrom. B. 861, 2:177-180

## Claims

1. Process for an industrial scale purification of bacteriophages intended for bacteriophage therapy, comprising the steps of:
- growing host bacteria of said bacteriophages;
- mixing bacteria and bacteriophages in order to allow the bacteriophages to grow;
- first filtering a preparation of mixed bacteria and bacteriophages, letting the bacteriophages pass through;
- first rotation-filtering, wherein the bacteriophages remain in solution in a retenate;
- second rotation-filtering with counterflow with respect to said first rotation-filtering, wherein the bacteriophages are subjected to elution; said second rotation-filtering with counterflow occurring at low pressures of 0.2 bar and with a slow rotation of 200 rotations per minute; and
- storing the solution of purified bacteriophages.

2. Process according to claim 1, **characterised in that** it further comprises, after said first filtering step, a second filtering step of the preparation of mixed bacteria and bacteriophages.

3. Process according to claim 1 or 2, **characterised in that** said first filtering step and/or said second filtering step are crossflow rotary filtering steps, said first filtering step being performed by using pores whose size is equal to 1000 nanometres, said second filtering step being performed by using pores whose size is equal to 200 nanometres.

4. Process according to claim 1, **characterised in that**, in said storing step, the solution of bacteriophages is subjected to spiral rotation or swirling adapted to obtain an homogeneous distribution of bacteriophages.

5. Process according to claim 1, **characterised in that** it further comprises the step of packaging the solution of bacteriophages in liquid form.

6. Process according to claim 1, **characterised in that** it further comprises the step of packaging the solution of bacteriophages in dry form after lyophilization.

7. System for an industrial scale purification of bacteriophages intended for bacteriophage therapy, comprising:
- a first fermenter (1), preferably of a high cell density chemiostatic type, adapted to perform a continuous growth of host bacteria;
- a second fermenter (3) connected to said first fermenter (1) and adapted to perform a mixing of bacteria and bacteriophages in order to allow the bacteriophages to grow;
**characterised in that** it further comprises:
- a filtering unit (5) connected to said second fermenter (3) and adapted to filter said preparation of bacteria and bacteriophages letting the bacteriophages pass through; and
- a rotary filtering unit (7) connected to said filtering unit (5) adapted to perform a first rotation-filtering, wherein the bacteriophages remain in solution in the retenate, and a second rotation-filtering with counterflow with respect to said first rotation filtering, wherein the bacteriophages are subjected to elution, said second rotation-filtering with counterflow occurring at low pressures of 0.2 bar and with a slow rotation of 200 rotations per minute.

8. System according to claim 7, **characterised in that** it further comprises:
- a first pump unit (42) connected to said rotary filtering unit (7) and adapted to pump an elution solution through the rotary filtering unit (7), retaining the bacteriophages in the retenate;
- an intermediate storage container (14) for the solution of purified bacteriophages; and
- a second pump unit (16) connected to said rotary filtering unit (7) and adapted to pump a cleaning solution through said rotary filtering unit (7) into said intermediate storage container (14).

9. System according to claim 7, **characterised in that** it further comprises:
- a first pump unit (10) connected to said rotary filtering unit (7) and adapted to pump an elution solution of said bacteriophages in the retenate;
- an intermediate storage container (14) for the solution of purified bacteriophages;
- a second pump unit (42) connected to said rotary filtering unit (7) and to said intermediate container (14) and adapted to pump a cleaning solution into said rotary filtering unit (7);
- a third pump unit (16) connected to said rotary filtering unit (7) and adapted to pump into said solution of bacteriophages, a counterflow solution, and then to pump both solutions into said rotary filtering unit (7) for the counterflow in an opposite direction; and
- optionally, a fourth pump unit (28) connected to said rotary filtering unit (7) and adapted to pump the final solution of clean bacteriophages into said intermediate storage container (14).

10. System according to claim 9, **characterised in that**, in said intermediate storage container (14), the obtained solution is subjected to rotation or swirling to obtain a homogeneous and purified distribution of bacteriophages.

11. System according to claim 7, **characterised in that** said filtering unit (5) has pores whose size is equal to 1000 nanometres, and said system comprises a further filtering unit (9) containing pores whose size is equal to 200 nanometres.

12. System according to claim 11, **characterised in that** said filtering unit (5) and said further filtering unit (9) are of the rotary type.

13. System according to claim 7, **characterised in that** said rotary filtering unit (7) is divided into two identical, separate rotary filtering units (7A, 7B), the first one (7A) of said separate rotary filtering units (7A, 7B) being connected to said filtering unit (5) and being adapted to perform a first rotation-filtering, wherein the bacteriophages remain in solution in the retenate, the second one (7B) of said separate rotary filtering units (7A, 7B) being connected to said first rotary filtering unit (7A) and being adapted to perform a second rotation-filtering with counterflow with respect to said first rotary filtering unit (7A), wherein the bacteriophages are subjected to elution and the obtained solution is subjected to rotation or swirling to obtain a homogeneous and purified distribution of bacteriophages.

14. System according to claim 7, **characterised in that** said rotary filtering unit (7) is adapted to firstly perform a first rotation-filtering, wherein the bacteriophages remain in solution in the retenate, and afterwards is adapted to separately perform a second rotation-filtering with counterflow with respect to said first rotation-filtering, wherein the bacteriophages are subjected to elution and the obtained solution is subjected to rotation or swirling to obtain a homogeneous and purified distribution of bacteriophages.

15. System according to claim 7, **characterised in that** said rotary filtering unit (7) is adapted to be detached, from one of said systems and said detached rotary filtering unit (7) is adapted to be inserted in another one of said systems.

## Patentansprüche

1. Verfahren zur großtechnischen Aufreinigung von für eine Bakteriophagentherapie vorgesehenen Bakteriophagen, das die folgenden Schritte umfasst:
- Züchten von Wirtsbakterien der Bakteriophagen;
- Mischen von Bakterien und Bakteriophagen, um es den Bakteriophagen zu ermöglichen, zu wachsen;
- erstes Filtern eines Präparats aus gemischten Bakterien und Bakteriophagen, wobei die Bakteriophagen durchlaufen gelassen werden;
- erstes Drehfiltern, wobei die Bakteriophagen in einem Retentat gelöst bleiben;
- zweites Drehfiltern im Gegenstrom in Bezug auf das erste Drehfiltern, wobei die Bakteriophagen einer Elution unterzogen werden; wobei das zweite Drehfiltern im Gegenstrom bei niedrigen Drücken von 0,2 Bar und mit einer langsamen Drehung mit 200 Umdrehungen pro Minute erfolgt; und
- Lagern der Lösung von aufgereinigten Bakteriophagen.

2. Verfahren nach Anspruch 1, **dadurch gekennzeichnet, dass** es überdies nach dem ersten Schritt des Filterns einen zweiten Schritt des Filterns des Präparats aus gemischten Bakterien und Bakteriophagen umfasst.

3. Verfahren nach Anspruch 1 oder 2, **dadurch gekennzeichnet, dass** der erste Schritt des Filterns und/oder der zweite Schritt des Filterns Querstrom-Drehfilterschritte sind, wobei der erste Schritt des Filterns unter Verwendung von Poren durchgeführt wird, deren Größe gleich 1.000 Nanometer ist, wobei der zweite Schritt des Filterns unter Verwendung von Poren durchgeführt wird, deren Größe gleich 200 Nanometer ist.

4. Verfahren nach Anspruch 1, **dadurch gekennzeichnet, dass** die Lösung von Bakteriophagen im Schritt des Lagerns einer Spiraldrehung oder einem Wirbeln unterzogen wird, die/das angepasst ist, um eine homogene Verteilung von Bakteriophagen zu erhalten.

5. Verfahren nach Anspruch 1, **dadurch gekennzeichnet, dass** es überdies den Schritt des Verpackens der Lösung von Bakteriophagen in flüssiger Form umfasst.

6. Verfahren nach Anspruch 1, **dadurch gekennzeichnet, dass** es überdies den Schritt des Verpackens der Lösung von Bakteriophagen in trockener Form nach Gefriertrocknung umfasst.

7. System zur großtechnischen Aufreinigung von für eine Bakteriophagentherapie vorgesehenen Bakteriophagen, das Folgendes umfasst:
- einen ersten Fermenter (1), vorzugsweise von der chemostatischen Art mit hoher Zelldichte, der angepasst ist, um ein kontinuierliches Wachstum von Wirtsbakterien durchzuführen;
- einen zweiten Fermenter (3), der mit dem ersten Fermenter (1) verbunden ist und angepasst ist, um ein Mischen von Bakterien und Bakteriophagen durchzuführen, um es den Bakteriophagen zu erlauben, zu wachsen;
**dadurch gekennzeichnet, dass** es überdies Folgendes umfasst:
- eine Filtereinheit (5), die mit dem zweiten Fermenter (3) verbunden ist und angepasst ist, um das Präparat von Bakterien und Bakteriophagen zu filtern und die Bakteriophagen durchlaufen zu lassen; und
- eine Drehfiltereinheit (7), die mit der Filtereinheit (5) verbunden ist und angepasst ist, um ein erstes Drehfiltern, wobei die Bakteriophagen in dem Retentat gelöst bleiben, und ein zweites Drehfiltern im Gegenstrom in Bezug auf das erste Drehfiltern durchzuführen, wobei die Bakteriophagen einer Elution unterzogen werden, wobei das zweite Drehfiltern im Gegenstrom bei niedrigen Drücken von 0,2 Bar und mit einer langsamen Drehung mit 200 Umdrehungen pro Minute erfolgt.

8. System nach Anspruch 7, **dadurch gekennzeichnet, dass** es überdies Folgendes umfasst:
- eine erste Pumpeneinheit (42), die mit der Drehfiltereinheit (7) verbunden ist und angepasst ist, um eine Elutionslösung durch die Drehfiltereinheit (7) zu pumpen, wobei die Bakteriophagen in dem Retentat gehalten werden;
- einen Zwischenlagerungsbehälter (14) für die Lösung von aufgereinigten Bakteriophagen; und
- eine zweite Pumpeneinheit (16), die mit der Drehfiltereinheit (7) verbunden ist und angepasst ist, um eine Reinigungslösung durch die Drehfiltereinheit (7) in den Zwischenlagerungsbehälter (14) zu pumpen.

9. System nach Anspruch 7, **dadurch gekennzeichnet, dass** es überdies Folgendes umfasst:
- eine erste Pumpeneinheit (10), die mit der Drehfiltereinheit (7) verbunden ist und angepasst ist, um eine Elutionslösung von den Bakteriophagen in dem Retentat zu pumpen;
- einen Zwischenlagerungsbehälter (14) für die Lösung von aufgereinigten Bakteriophagen;
- eine zweite Pumpeneinheit (42), die mit der Drehfiltereinheit (7) und dem Zwischenlagerungsbehälter (14) verbunden ist und angepasst ist, um eine Reinigungslösung in die Drehfiltereinheit (7) zu pumpen;
- eine dritte Pumpeneinheit (16), die mit der Drehfiltereinheit (7) verbunden ist und angepasst ist, um eine Gegenstromlösung in die Lösung von Bakteriophagen zu pumpen und dann beide Lösungen für den Gegenstrom in einer entgegengesetzten Richtung in die Drehfiltereinheit (7) zu pumpen; und
- wahlfrei eine vierte Pumpeneinheit (28), die mit der Drehfiltereinheit (7) verbunden ist und angepasst ist, um die Endlösung von gereinigten Bakteriophagen in den Zwischenlagerungsbehälter (14) zu pumpen.

10. System nach Anspruch 9, **dadurch gekennzeichnet, dass** die erhaltene Lösung in dem Zwischenlagerungsbehälter (14) einer Drehung oder einem Wirbeln unterzogen wird, um eine homogene und aufgereinigte Verteilung von Bakteriophagen zu erhalten.

11. System nach Anspruch 7, **dadurch gekennzeichnet, dass** die Filtereinheit (5) Poren aufweist, deren Größe gleich 1.000 Nanometer ist, und das System eine weitere Filtereinheit (9) umfasst, die Poren enthält, deren Größe gleich 200 Nanometer ist.

12. System nach Anspruch 11, **dadurch gekennzeichnet, dass** die Filtereinheit (5) und die weitere Filtereinheit (9) von der drehbaren Art sind.

13. System nach Anspruch 7, **dadurch gekennzeichnet, dass** die Drehfiltereinheit (7) in zwei identische getrennte Drehfiltereinheiten (7A, 7B) unterteilt ist, wobei die erste (7A) der getrennten Drehfiltereinheiten (7A, 7B) mit der Filtereinheit (5) verbunden ist und angepasst ist, um ein erstes Drehfiltern durchzuführen, wobei die Bakteriophagen in dem Retentat gelöst bleiben, wobei die zweite (7B) der getrennten Drehfiltereinheiten (7A, 7B) mit der ersten Drehfiltereinheit (7A) verbunden ist und angepasst ist, um ein zweites Drehfiltern im Gegenstrom in Bezug auf die erste Drehfiltereinheit (7A) durchzuführen, wobei die Bakteriophagen einer Elution unterzogen werden und die erhaltene Lösung einer Drehung oder einem Wirbeln unterzogen wird, um eine homogene und aufgereinigte Verteilung von Bakteriophagen zu erhalten.

14. System nach Anspruch 7, **dadurch gekennzeichnet, dass** die Drehfiltereinheit (7) angepasst ist, um zuerst eine erste Drehfilterung durchzuführen, wobei die Bakteriophagen in dem Retentat gelöst bleiben, und angepasst ist, um danach ein zweites Drehfiltern im Gegenstrom in Bezug auf das erste Drehfiltern durchzuführen, wobei die Bakteriophagen einer Elution unterzogen werden und die erhaltene Lösung einer Drehung oder einem Wirbeln unterzogen wird, um eine homogene und aufgereinigte Verteilung von Bakteriophagen zu erhalten.

15. System nach Anspruch 7, **dadurch gekennzeichnet, dass** die Drehfiltereinheit (7) angepasst ist, um von einem der Systeme abgenommen zu werden und die abgenommene Drehfiltereinheit (7) angepasst ist, um in ein anderes der Systeme eingesetzt zu werden.

## Revendications

1. Procédé de purification à l'échelle industrielle de bactériophages destinés à une thérapie par bactériophages, comprenant les étapes de :
- croissance de bactéries hôtes desdits bactériophages ;
- mélange de bactéries et bactériophages afin de permettre aux bactériophages de croître ;
- première filtration d'une préparation de bactéries et bactériophages mélangés, laissant les bactériophages passer à travers ;
- première filtration centrifuge, dans laquelle les bactériophages restent en solution dans un rétentat ;
- seconde filtration centrifuge avec contre-courant par rapport à ladite première filtration centrifuge, dans laquelle les bactériophages sont soumis à une élution ; ladite seconde filtration centrifuge avec contre-courant se produisant à de basses pressions de 0,2 bar et avec une rotation lente de 200 tours par minute ; et
- stockage de la solution de bactériophages purifiés.

2. Procédé selon la revendication 1, **caractérisé en ce qu'**il comprend en outre, après ladite première étape de filtration, une seconde étape de filtration de la préparation de bactéries et bactériophages mélangés.

3. Procédé selon la revendication 1 ou 2, **caractérisé en ce que** ladite première étape de filtration et/ou ladite seconde étape de filtration sont des étapes de filtration centrifuge à écoulement transversal, ladite première étape de filtration étant réalisée en utilisant des pores dont la taille est égale à 1000 nanomètres, ladite seconde étape de filtration étant réalisée en utilisant des pores dont la taille est égale à 200 nanomètres.

4. Procédé selon la revendication 1, **caractérisé en ce que**, dans ladite étape de stockage, la solution de bactériophages est soumise à une rotation en spirale ou un tourbillonnement adapté(e) à obtenir une distribution homogène de bactériophages.

5. Procédé selon la revendication 1, **caractérisé en ce qu'**il comprend en outre l'étape d'emballage de la solution de bactériophages sous forme liquide.

6. Procédé selon la revendication 1, **caractérisé en ce qu'**il comprend en outre l'étape d'emballage de la solution de bactériophages sous forme sèche après lyophilisation.

7. Système de purification à l'échelle industrielle de bactériophages destinés à une thérapie par bactériophages, comprenant :
- un premier fermenteur (1), de préférence d'un type chimiostatique à haute densité cellulaire, adapté à réaliser une croissance continue de bactéries hôtes ;
- un second fermenteur (3) connecté audit premier fermenteur (1) et adapté à réaliser un mélange de bactéries et bactériophages afin de permettre aux bactériophages de croître ;
**caractérisé en ce qu'**il comprend en outre :
- un module de filtration (5) connecté audit second fermenteur (3) et adapté à filtrer ladite préparation de bactéries et bactériophages, laissant les bactériophages passer à travers ; et
- un module de filtration centrifuge (7) connecté audit module de filtration (5) adapté à réaliser une première filtration centrifuge, dans laquelle les bactériophages restent en solution dans le rétentat, et une seconde filtration centrifuge avec contre-courant par rapport à ladite première filtration centrifuge, dans laquelle les bactériophages sont soumis à une élution, ladite seconde filtration centrifuge avec contre-courant se produisant à de basses pressions de 0,2 bar et avec une rotation lente de 200 tours par minute.

8. Système selon la revendication 7, **caractérisé en ce qu'**il comprend en outre :
- un premier module de pompe (42) connecté audit module de filtration centrifuge (7) et adapté à pomper une solution d'élution à travers le module de filtration centrifuge (7), retenant les bactériophages dans le rétentat ;
- un récipient de stockage intermédiaire (14) pour la solution de bactériophages purifiés ; et
- un second module de pompe (16) connecté audit module de filtration centrifuge (7) et adapté à pomper une solution de nettoyage à travers ledit module de filtration centrifuge (7) dans ledit récipient de stockage intermédiaire (14).

9. Système selon la revendication 7, **caractérisé en ce qu'**il comprend en outre :
- un premier module de pompe (10) connecté audit module de filtration centrifuge (7) et adapté à pomper une solution d'élution desdits bactériophages dans le rétentat ;
- un récipient de stockage intermédiaire (14) pour la solution de bactériophages purifiés ;
- un deuxième module de pompe (42) connecté audit module de filtration centrifuge (7) et audit récipient intermédiaire (14) et adapté à pomper une solution de nettoyage dans ledit module de filtration centrifuge (7) ;
- un troisième module de pompe (16) connecté audit module de filtration centrifuge (7) et adapté à pomper dans ladite solution de bactériophages, une solution à contre-courant, puis à pomper les deux solutions dans ledit module de filtration centrifuge (7) pour le contre-courant dans une direction opposée ; et en option, un quatrième module de pompe (28) connecté audit module de filtration centrifuge (7) et adapté à pomper la solution finale de bactériophages propres dans ledit récipient de stockage intermédiaire (14).

10. Système selon la revendication 9, **caractérisé en ce que**, dans ledit récipient de stockage intermédiaire (14), la solution obtenue est soumise à une rotation ou un tourbillonnement pour obtenir une distribution homogène et purifiée de bactériophages.

11. Système selon la revendication 7, **caractérisé en ce que** ledit module de filtration (5) a des pores dont la taille est égale à 1000 nanomètres, et ledit système comprend un autre module de filtration (9) contenant des pores dont la taille est égale à 200 nanomètres.

12. Système selon la revendication 11, **caractérisé en ce que** ledit module de filtration (5) et ledit autre module de filtration (9) sont de type centrifuge.

13. Système selon la revendication 7, **caractérisé en ce que** ledit module de filtration centrifuge (7) est divisé en deux modules de filtration centrifuge séparés identiques (7A, 7B), le premier (7A) desdits modules de filtration centrifuge séparés (7A, 7B) étant connecté audit module de filtration (5) et étant adapté à réaliser une première filtration centrifuge, dans laquelle les bactériophages restent en solution dans le rétentat, le second (7B) desdits modules de filtration centrifuge séparés (7A, 7B) étant connecté audit premier module de filtration centrifuge (7A) et étant adapté à réaliser une seconde filtration centrifuge avec contre-courant par rapport audit premier module de filtration centrifuge (7A), dans laquelle les bactériophages sont soumis à une élution et la solution obtenue est soumise à une rotation ou un tourbillonnement pour obtenir une distribution homogène et purifiée de bactériophages.

14. Système selon la revendication 7, **caractérisé en ce que** ledit module de filtration centrifuge (7) est adapté à réaliser dans un premier temps une première filtration centrifuge, dans laquelle les bactériophages restent en solution dans le rétentat, et est ensuite adapté à réaliser séparément une seconde filtration centrifuge avec contre-courant par rapport à ladite première filtration centrifuge, dans laquelle les bactériophages sont soumis à une élution et la solution obtenue est soumise à une rotation ou un tourbillonnement pour obtenir une distribution homogène et purifiée de bactériophages.

15. Système selon la revendication 7, **caractérisé en ce que** ledit module de filtration centrifuge (7) est adapté à être déconnecté d'un desdits systèmes et ledit module de filtration centrifuge déconnecté (7) est adapté à être inséré dans un autre desdits systèmes.
